# EUROPEAN PATENT APPLICATION

(11) **EP 0 753 291 A1**
(43) Date of publication of application: **15.01.1997**
(21) Application number: 95935459.8
(22) Date of filing: 31.10.1995
(51) Int. Cl.: A61F 11/00, A61F 13/38

(54) **HYGIENIC STICK FOR EARS' CLEANING**

(30) Priority: 04.11.1994 ES 9402805 U
(71) Applicant: Serra Caselles, Genoveva Maria, 3750 Pedreguer (ES)
(72) Inventor: Serra Caselles, Genoveva Maria, 3750 Pedreguer (ES)
(74) Representative: Elzaburu Marquez, Alberto
(86) International application number: ES9500119
(87) International publication number: WO9614033

(57) **Abstract**

It consists of an elongate structure (1) which has, at its upper end, a head with a curved encircling shape derived from the actual body of the wand with an internal orifice (2), the encircling surface being covered with a soft material (3).

## Description

### SUBJECT OF THE INVENTION

The subject matter of the present invention is a hygienic wand which can be applied to the auditory canal for the purpose of cleaning wax from the ears, which has as its main characteristic its ease of handling and its improved accessibility, being non-harmful when used.

### BACKGROUND OF THE INVENTION

The wands usually used to clean wax from the ears in order to improve the user's hygiene conditions as well as his hearing capacity are known, there being, at one of the ends of the wand, a termination in the form of a cotton swab which is used inside the ear by pressing on the walls of the ear which have wax adhering to them.

This type of wand has the disadvantage that, when it touches the ear, it causes pain in the ear and it is not possible to completely clean the auditory canal.

Normally, the hygienic wand employed has an elongate surface with two tapered ends, a rounded mass of cotton being arranged at one of its ends.

To date, a type of instrument with another configuration or a structure which is different from that mentioned above and which makes it possible to carry out the cleaning tasks required of these wands while maintaining conditions of accessibility has not been envisaged.

### DESCRIPTION OF THE INVENTION

The present model consists of an elongate wand which can be gripped at one of its ends, having, at the other end, a head of encircling shape which is curved on itself and is made up of the same material as the body of the wand, delimiting an internal orifice.

The walls which define this orifice are normally covered with a soft material to offset the sharp effect of the wand on the ear.

The fact that it has an orifice provides easier access to the particles of wax, dislodging them without producing any kind of pain.

Unlike the method used previously, in this case no pressure is applied to the particles of wax adhering to the walls, the latter being scraped off.

The body of the wand may be composed of different sections which enable it to be held when it is inserted inside the ear in order to clean the latter.

### DESCRIPTION OF THE DRAWINGS

To supplement the description which is being given and in order to assist better understanding of the characteristics of the invention, the present specification is accompanied, as an integral part thereof, by a set of plans which, by way of non-limiting illustration, show the following:

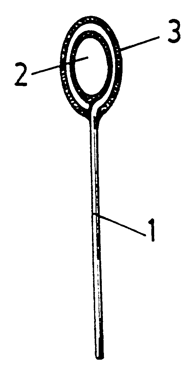

Figure 1. - shows a view of the hygienic wand for ears which is covered on its head.

### PREFERRED EMBODIMENT OF THE INVENTION

An examination of the figure shown reveals that the hygienic wand consists of an elongate surface (1) to facilitate its accessibility inside the ear, which consists, at its upper end, of a head with a curved encircling shape inside which there is an orifice (2), in this way facilitating, by means of this end, scraping-off of the particles of wax adhering to the auditory canal.

To prevent the excessive fineness of the wand causing damage inside the ear, the upper encircling part making up the head is covered with a soft material (3).

It is not considered necessary to expand this description for any expert in the field to understand the scope of the invention and the advantages deriving therefrom.

The materials, shape, size and arrangement of the elements may be varied provided they do not alter the essential nature of the invention.

The terms in which this specification has been described must always be taken in their widest and non-limiting sense.

## Claims

1. Hygienic wand for cleaning the ears, essentially characterized in that it consists of an elongate structure which has, at its upper end, a head with a curved encircling shape derived from the actual body of the wand with an internal orifice, the encircling surface being covered with a soft material.

2. Hygienic wand for cleaning the ears.
